# EUROPEAN PATENT APPLICATION

(11) **EP 4 442 780 A1**
(43) Date of publication of application: **09.10.2024**
(21) Application number: 23167191.8
(22) Date of filing: 07.04.2023
(51) Int. Cl.: C09D 183/04, A61M 5/31, A61M 5/315

(54) **LOW TEMPERATURE STORAGE MEDICAL CONTAINER**

(71) Applicant: Becton, Dickinson and Company, Franklin Lakes, NJ 07417-1880 (US)
(72) Inventor: RODRIGUEZ SAN JUAN, Nestor, Hamburg, New Jersey 07419 (US); PERRIN, Eloïse, 38320 Eybens (FR)
(74) Representative: Germain Maureau

(57) **Abstract**

A low temperature storage medical container is provided. The low temperature storage medical container includes a barrel including an inner surface and a stopper in gliding arrangement with at least a portion of the inner surface of the barrel. At least one of the inner surface of the barrel and the stopper are at least partially coated with a lubricant composition, the lubricant composition including at least one of a compound of Formula (I), a compound of Formula (II), or combinations thereof. A method of reducing crystallization of a lubricant coating on a low temperature storage medical container during freezing and thawing is also provided.

## Description

### BACKGROUND OF THE INVENTION

### Field of the Invention

The present disclosure relates to a low temperature storage medical container and a method of reducing crystallization of a lubricant coating on a low temperature storage medical container during freezing and thawing.

### Technical Background

The use of medical containers for the storage of vaccines or cell therapies require low storage temperatures, for examples at temperatures of less than -40 degrees Celsius (°C). These medical containers are first cooled to low temperatures (e.g., -40°C), stored, and then brought back to room temperature (e.g., 25°C) through a thawing process.

During this freeze and thaw thermal cycle, lubricants (e.g., polydimethylsiloxane (PDMS) silicone oil) and rubber (e.g., butyl rubber) used in the medical containers may cross their phase transitions. Depending on the rate of the temperature change, the whole system is not in equilibrium, but rather, heat is continuously being drawn or given to the system by the surroundings, such as through cooling or heating systems, respectively.

In medical containers that include a stopper, the stopper re-conforms to the glass surface of the container at each moment of cooling or heating. However, this re-conforming process of the stopper is limited by the thermal and elastic energy available during the thermal cycling. The stopper re-conforming to container closure integrity (CCI) is needed due to either the differential thermal expansion between the stopper and the glass and/or any displacement caused by the differential pressures between the inside and the outside the medical container. The freeze and/or thaw processes of the stopper are different from either a stress relaxation or an insertion at a fixed temperature when the system is in thermal equilibrium.

The crossing of the phase transitions involves a change in heat capacity, as well as specific volume, which is a second order thermodynamic transition. In addition to a differential coefficient of thermal expansion between the components (e.g., a glass or polymer barrel, stopper, and lubricant), the abrupt and uncontrolled change of specific volume during the phase transition and the rigidity of the material (glassy or crystalline) poses a risk to CCI.

There is a need for a medical container for low temperature storage that uses a lubricant that does not crystalize upon freezing and thawing in order to eliminate or mitigate the challenge to CCI due to thermal transitions. The low temperature storage medical container of the present disclosure is configured to address these issues.

### SUMMARY OF THE INVENTION

In some non-limiting examples or aspects of the present disclosure, provided is a low temperature storage medical container. The low temperature storage medical container includes: a barrel having an inner surface; and a stopper in gliding arrangement with at least a portion of the inner surface of the barrel, wherein at least one of the inner surface of the barrel and the stopper are at least partially coated with a lubricant composition, the lubricant composition including at least one of a compound of Formula (I): 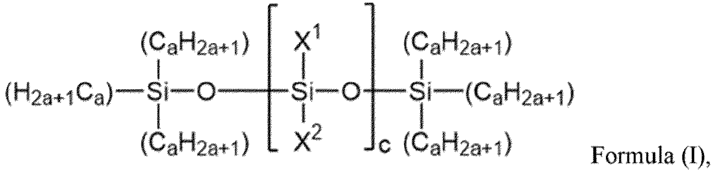 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1}, wherein b is different from a, and wherein c is an integer greater than or equal to 1; a compound of Formula (II): 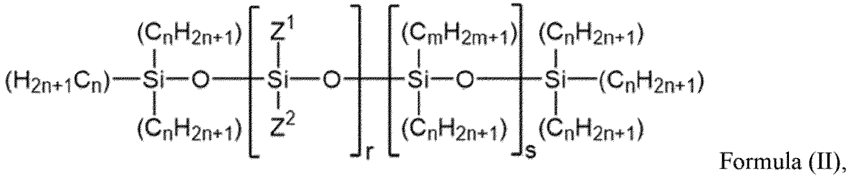 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥH₂ᵥ₊₁, wherein p and t are each independently integers greater or equal to 1, wherein v is an integer that is different from n, wherein when Z² is CₙH₂ₙ₊₁, Z¹ is phenyl, CₚH₂ₚCH(CₜH₂ₜ₊₁)phenyl, or CᵥH₂ᵥ₊₁ wherein v is different from n, and wherein r and s are each independently an integer greater than or equal to 1; or combinations thereof.

In some non-limiting examples or aspects of the present disclosure, provided is a method of reducing crystallization of a lubricant coating on a low temperature storage medical container during freezing and thawing. The medical container comprises: a barrel having an inner surface; and a stopper in gliding arrangement with at least a portion of the inner surface of the barrel. The method includes: coating a lubricant composition on at least one of the inner surface of the barrel and the stopper, the lubricant composition including at least one of: a compound of Formula (I): 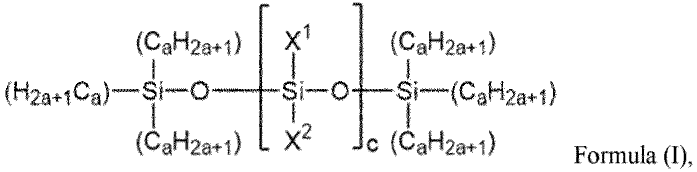 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, and wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1} wherein b is different from a, and wherein c is an integer greater than or equal to 1; a compound of Formula (II): 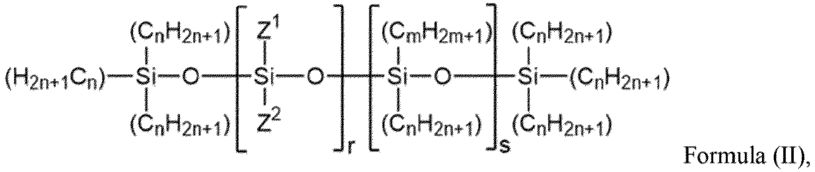 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥF₂ᵥ₊₁, wherein p and t are each independently integers greater or equal to 1, wherein v is an integer that is different from n, and wherein when Z² is CₙH₂ₙ₊₁, Z¹ is phenyl, CₚH₂ₚCH(CₜH₂ₜ₊₁)phenyl, or CᵥF₂ᵥ₊₁, wherein v is different from n, and wherein r and s are each independently an integer greater than or equal to 1; or combinations thereof.

A low temperature storage medical container and a method of reducing crystallization of a lubricant coating on a low temperature storage medical container during freezing and thawing may be characterized by one or more of the following aspects.

In a first aspect, a low temperature storage medical container, includes: a barrel including an inner surface; and a stopper in gliding arrangement with at least a portion of the inner surface of the barrel, wherein at least one of the inner surface of the barrel and the stopper are at least partially coated with a lubricant composition, the lubricant composition including at least one of: a compound of Formula (I): 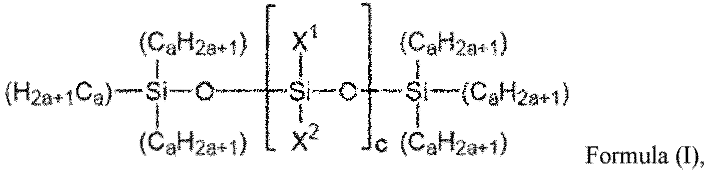 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, and wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1} wherein b is different from a, and wherein c is an integer greater than or equal to 1; a compound of Formula (II): 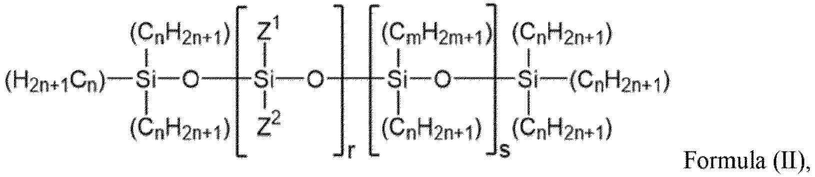 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥF₂ᵥ₊₁, wherein p and t are each independently integers greater or equal to 1, wherein v is an integer that is different from n, and wherein when Z² is CₙH₂ₙ₊₁, Z¹ is phenyl, CₚH₂ₚCH(CₜH₂ₜ₊₁)phenyl, or CᵥF₂ᵥ₊₁, wherein v is different from n, and wherein r and s are each independently an integer greater than or equal to 1; or combinations thereof.

In a second aspect, in the low temperature storage medical container in accordance with the first aspect, the lubricant composition further includes one or more small molecule surfactants, one or more an anti-nucleation agents, or combinations thereof.

In a third aspect, in the low temperature storage medical container in accordance with the first aspect or the second aspect, the one or more small molecule surfactants are non-ionic small molecule surfactants.

In a fourth aspect, in the low temperature storage medical container in accordance with any one of the first aspect to the third aspect, the non-ionic surfactant includes polyoxyethylene sorbitan monolaurate.

In a fifth aspect, in the low temperature storage medical container in accordance with any one of the first aspect to the fourth aspect, the lubricant composition includes the compound of Formula (I) wherein both X¹ and X² comprise C_{b}H_{2b+1} and a and b are greater than or equal to 2 and less than or equal to 10, such as greater than or equal to 2 and less than or equal to 5.

In a sixth aspect, in the low temperature storage medical container in accordance with any one of the first aspect to the fifth aspect, the lubricant composition includes: 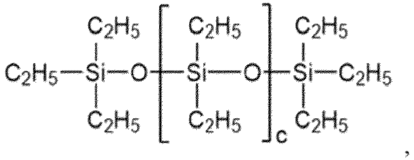 wherein c is greater than or equal to 1.

In a seventh aspect, in the low temperature storage medical container in accordance with any one of the first aspect to the sixth aspect, the lubricant composition includes the compound of Formula (I), wherein X¹ comprises C_{d}H_{2d}CH(C_{f}H_{2f+1})phenyl and X² comprises CₐH₂ₐ₊₁, where a, d, c, and f are each independently greater than or equal to 1.

In an eighth aspect, in the low temperature storage medical container in accordance with any one of the first aspect to the seventh aspect, the lubricant composition includes: 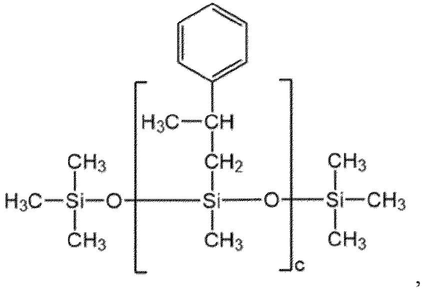 wherein c is greater than or equal to 1.

In a ninth aspect, in the low temperature storage medical container in accordance with any one of the first aspect to the eighth aspect, the lubricant composition includes the compound of Formula (II) wherein Z¹ and Z² comprise phenyl and m and n are each independently greater than or equal to 1.

In a tenth aspect, in the low temperature storage medical container in accordance with any one of the first aspect to the ninth aspect, the lubricant composition includes: 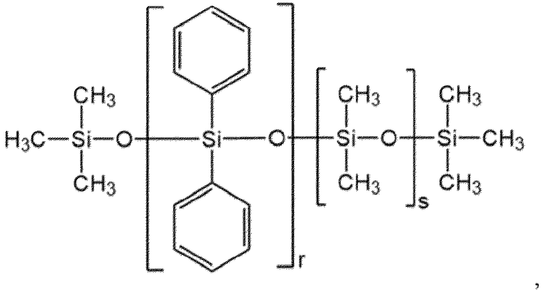 wherein r and s are each independently greater than or equal to 1.

In an eleventh aspect, in the low temperature storage medical container in accordance with any one of the first aspect to the tenth aspect, the lubricant composition includes the compound of Formula (II), wherein: Z¹ comprises CₚH₂ₚCH(CtH₂ₜ₊₁)phenyl wherein p and t are each independently integers greater or equal to 1, Z² comprises CₙH₂ₙ₊₁ wherein n is 1, m is greater than or equal to 1, and r and s are each independently an integer greater than or equal to 1.

In a twelfth aspect, in the low temperature storage medical container in accordance with any one of the first aspect to the eleventh aspect, m is greater than or equal to 2 and less than or equal to 12, such as greater than or equal to 2 and less than or equal to 8.

In a thirteenth aspect, in the low temperature storage medical container in accordance with any one of the first aspect to the twelfth aspect, the lubricant composition includes: 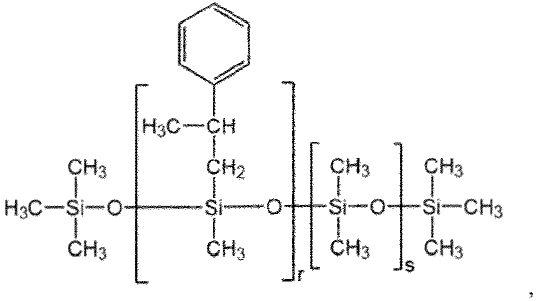 wherein r and s are each independently an integer greater than or equal to 1.

In a fourteenth aspect, in the low temperature storage medical container in accordance with any one of the first aspect to the thirteenth aspect or the twelfth aspect, the lubricant composition includes: 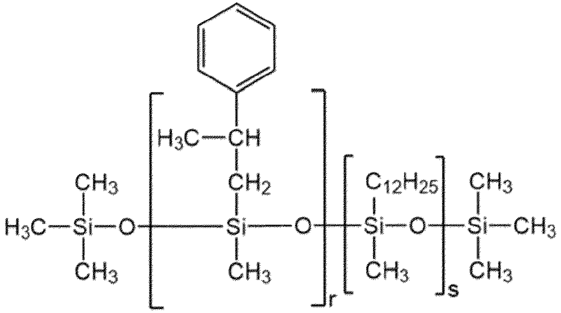 wherein r and s are each independently an integer greater than or equal to 1.

In a fifteenth aspect, in the low temperature storage medical container in accordance with any one of the first aspect to the fourteenth aspect or the twelfth aspect, the lubricant composition includes: 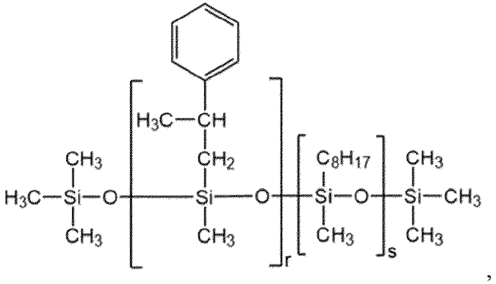 wherein r and s are each independently an integer greater than or equal to 1.

In a sixteenth aspect, in the low temperature storage medical container in accordance with any one of the first aspect to the fifteenth aspect, the lubricant composition includes: 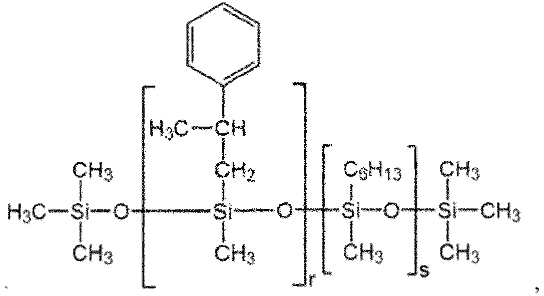 wherein r and s are each independently an integer greater than or equal to 1.

In a seventeenth aspect, in the low temperature storage medical container in accordance with any one of the first aspect to the sixteenth aspect to the sixteenth aspect, the compound of Formula (II) includes from at least 45% to at least 85% of 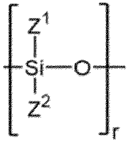 and from at least 15% to at least 55% of

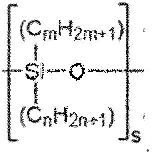

In an eighteenth aspect, in the low temperature storage medical container in accordance with any one of the first aspect to the seventeenth aspect, the lubricant composition includes a viscosity of about 200 centistokes (cSt) to about 5,000 cSt or such as about 500 cSt to about 2,000 cSt.

In a nineteenth aspect, in the low temperature storage medical container in accordance with any one of the first aspect to the eighteenth aspect, an average lubricant composition thickness to surface roughness of at least one of the inner surface of the barrel and the stopper ratio is between 0.001 and 3.0.

In a twentieth aspect, in the low temperature storage medical container in accordance with any one of the first aspect to the nineteenth aspect, the lubricant composition has a coating thickness of at least 1.0 nanometers (nm).

In a twenty-first aspect, in the low temperature storage medical container in accordance with any one of the first aspect to the twentieth aspect, the barrel is made of glass or plastic.

In a twenty-second aspect, in the low temperature storage medical container in accordance with any one of the first aspect to the twenty-first aspect, the barrel is made of glass.

In a twenty-third aspect, in the low temperature storage medical container in accordance with any one of the first aspect to the twenty-second aspect, the stopper includes a rubber selected from the group consisting of butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, natural rubbers, and combinations thereof.

In a twenty-fourth aspect, in the in the low temperature storage medical container in accordance with any one of the first aspect to the twenty-third aspect, the stopper includes butyl rubber.

In a twenty-fifth aspect, in the low temperature storage medical container in accordance with any one of the first aspect to the twenty-fourth aspect, the stopper includes an elastomeric copolymer selected from the group consisting of including: thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS), and combinations thereof.

In a twenty-sixth aspect, in the low temperature storage medical container in accordance with any one of the first aspect to the twenty-fifth aspect, the stopper is a coated stopper or an uncoated stopper.

In a twenty-seventh aspect, in the low temperature storage medical container in accordance with any one of the first aspect to the twenty-sixth aspect, the stopper is a coated stopper.

In a twenty-eighth aspect, in the low temperature storage medical container in accordance with any one of the first aspect to the twenty-seventh aspect, the stopper is a coated with polytetrafluoroethylene.

In a twenty-ninth aspect, in the low temperature storage medical container in accordance with any one of the first aspect to the twenty-eighth aspect, the low temperature storage medical container includes a syringe including the barrel defining an interior, a plunger rod configured to displace the stopper, and an outlet in fluid communication with the interior of the barrel.

In a thirtieth aspect, in the low temperature storage medical container in accordance with any one of the first aspect to the twenty-ninth aspect, the outlet is a Luer tip or a needle.

In a thirty-first aspect, in the low temperature storage medical container in accordance with any one of the first aspect to the thirtieth aspect, the outlet is a needle.

In a thirty-second aspect, in the low temperature storage medical container in accordance with any one of the first aspect to the thirty-first aspect, the low temperature storage medical container includes a therapeutic composition.

In a thirty-third aspect, in the low temperature storage medical container in accordance with any one of the first aspect to the thirty-second aspect, the therapeutic composition includes a vaccine composition.

In a thirty-fourth aspect, in the low temperature storage medical container in accordance with any one of the first aspect to the thirty-third aspect, the therapeutic composition includes one or more cells.

In a thirty-fifth aspect, in the low temperature storage medical container in accordance with any one of the first aspect to the thirty-fourth aspect, the low temperature storage medical container is intended to be stored at temperatures of less than or equal to -60°C, such as less than or equal to -80°C, or such as less than or equal to -170°C.

In a thirty-sixth aspect, a method of reducing crystallization of a lubricant coating on a low temperature storage medical container during freezing and thawing includes: the medical container including: a barrel comprising an inner surface; and a stopper in gliding arrangement with at least a portion of the inner surface of the barrel, the method includes: coating a lubricant composition on at least one of the inner surface of the barrel and the stopper, the lubricant composition including at least one of: a compound of Formula (I): 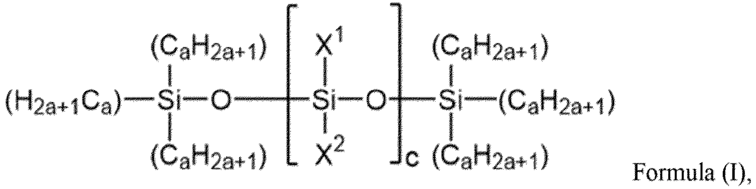 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, and wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1} wherein b is different from a, and wherein c is an integer greater than or equal to 1; a compound of Formula (II): 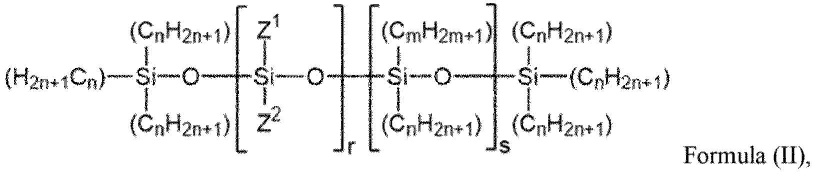 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥF₂ᵥ₊₁, wherein p and t are each independently integers greater or equal to 1, wherein v is an integer that is different from n, and wherein when Z² is CₙH₂ₙ₊₁, Z¹ is phenyl, CₚH₂ₚCH(CₜH₂ₜ₊₁)phenyl, or CᵥF₂ᵥ₊₁, wherein v is different from n, and wherein r and s are each independently an integer greater than or equal to 1; or combinations thereof.

In a thirty-seventh aspect, in the method in accordance with the thirty-sixth aspect, the lubricant composition further includes one or more small molecule surfactants, one or more an anti-nucleation agents, or combinations thereof.

In a thirty-eighth aspect, in the method in accordance with the thirty-sixth aspect or the thirty-seventh aspect, the one or more small molecule surfactants are non-ionic small molecule surfactants.

In a thirty-ninth aspect, in the method in accordance with any one of the thirty-sixth aspect to the thirty-eighth aspect, the non-ionic surfactant includes polyoxyethylene sorbitan monolaurate.

In a fortieth aspect, in the method in accordance with any one of the thirty-sixth aspect to the thirty-ninth aspect, the lubricant composition includes the compound of Formula (I) wherein both X¹ and X² comprise C_{b}H_{2b+1} and a and b are greater than or equal to 2 and less than or equal to 10, such as greater than or equal to 2 and less than or equal to 5.

In a forty-first aspect, in the method in accordance with any one of the thirty-sixth aspect to the fortieth aspect, the lubricant composition includes: 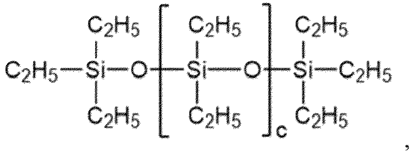 wherein c is greater than or equal to 1.

In a forty-second aspect, in the method in accordance with any one of the thirty-sixth aspect to the forty-first aspect, the lubricant composition includes the compound of Formula (I), wherein X¹ comprises C_{d}H_{2d}CH(C_{f}H_{2f+1})phenyl and X² comprises CₐH₂ₐ₊₁, where a, d, c, and f are each independently greater than or equal to 1.

In a forty-third aspect, in the method in accordance with any one of the thirty-sixth aspect to the forty-second aspect, the lubricant composition includes: 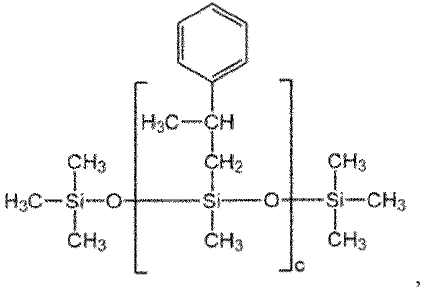 wherein c is greater than or equal to 1

In a forty-fourth aspect, in the method in accordance with any one of the thirty-sixth aspect to the forty-third aspect, the lubricant composition includes the compound of Formula (II) wherein Z¹ and Z² comprise phenyl and m and n are each independently greater than or equal to 1.

In a forty-fifth aspect, in the method in accordance with any one of the thirty-sixth aspect the forty-fourth aspect, the lubricant composition includes: 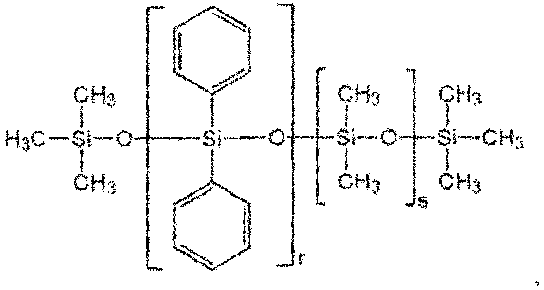 wherein r and s are each independently greater than or equal to 1.

In a forty-sixth aspect, in the method in accordance with any one of the thirty-sixth aspect to the forty-fifth aspect, the lubricant composition includes the compound of Formula (II), wherein: Z¹ comprises CₚH₂ₚCH(CtH₂ₜ₊₁)phenyl wherein p and t are each independently integers greater or equal to 1, Z² comprises CₙH₂ₙ₊₁ wherein n is 1, m is greater than or equal to 1, and r and s are each independently an integer greater than or equal to 1.

In a forty-seventh aspect, in the method in accordance with any one of the thirty-sixth aspect to the forty-sixth aspect, m is greater than or equal to 2 and less than or equal to 12, such as greater than or equal to 2 and less than or equal to 8.

In a forty-eighth aspect, in the method in accordance with any one of the thirty-sixth aspect to the forty-seventh aspect, the lubricant composition includes: 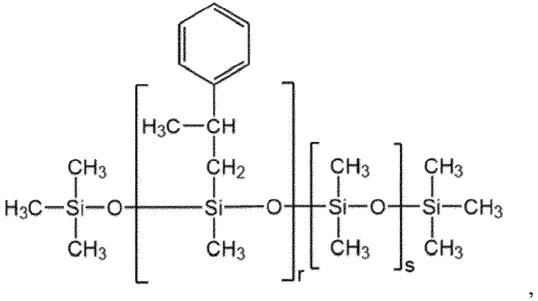 wherein r and s are each independently an integer greater than or equal to 1.

In a forty-ninth aspect, in the method in accordance with any one of the thirty-first aspect to the forty-eighth aspect, the lubricant composition includes: 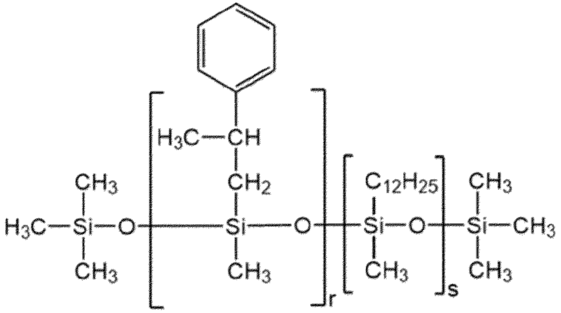 wherein r and s are each independently an integer greater than or equal to 1.

In a fiftieth aspect, in the method in accordance with any one of the thirty-sixth aspect to the forty-ninth aspect, the lubricant composition includes: 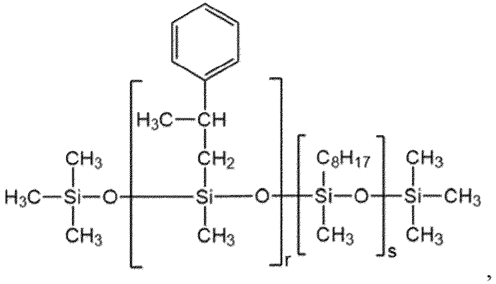 wherein r and s are each independently an integer greater than or equal to 1.

In a fifty-first aspect, in the method in accordance with any one of the thirty-sixth aspect the fiftieth aspect, the lubricant composition includes: 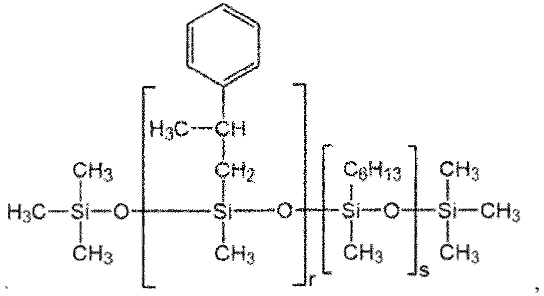 wherein r and s are each independently an integer greater than or equal to 1.

In a fifty-second aspect, in the method in accordance with any one of the thirty-sixth aspect to the fifty-first aspect, the compound of Formula (II) includes from at least 45% to at least 85% of 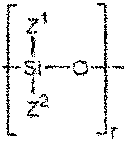 and from at least 15% to at least 55% of

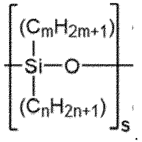

In a fifty-third aspect, in the method in accordance with any one of the thirty-sixth aspect to the fifty-second aspect, the lubricant composition includes a viscosity of about 200 centistokes (cSt) to about 5,000 cSt or such as about 500 cSt to about 2,000 cSt.

In a fifty-fourth aspect, in the method in accordance with any one of the thirty-sixth aspect to the fifty-third aspect, an average lubricant composition thickness to surface roughness of at least one of the inner surface of the barrel and the stopper ratio is between 0.001 and 3.0.

In a fifty-fifth aspect, in the method in accordance with any one of the thirty-sixth aspect to the fifty-fourth aspect, the lubricant composition has a coating thickness of at least 1.0 nanometers (nm).

In a fifty-sixth aspect, in the method in accordance with any one of the thirty-sixth aspect to the fifty-fifth aspect, the barrel is made of glass or plastic.

In a fifty-seventh aspect, in the method in accordance with any one of the thirty-sixth aspect to the fifty-sixth aspect, the barrel is made of glass.

In a fifty-eighth aspect, in the method in accordance with any one of the thirty-sixth aspect to the fifty-seventh aspect, the stopper includes a rubber selected from the group consisting of butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, natural rubbers, and combinations thereof.

In a fifty-ninth aspect, in the in the method in accordance with any one of the thirty-sixth aspect to the fifty-eighth aspect, the stopper includes butyl rubber.

In a sixtieth aspect, in the method in accordance with any one of the thirty-sixth aspect to the fifty-ninth aspect, the stopper includes an elastomeric copolymer selected from the group consisting of including: thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS), and combinations thereof.

In a sixty-first aspect, in the method in accordance with any one of the thirty-sixth aspect to the sixtieth aspect, the stopper is a coated stopper or an uncoated stopper.

In a sixty-second aspect, in the method in accordance with any one of the thirty-sixth aspect to the sixty-first aspect, the stopper is a coated stopper.

In a sixty-third aspect, in the method in accordance with any one of the thirty-sixth aspect to the sixty-second aspect, the stopper is a coated with polytetrafluoroethylene.

In a sixty-fourth aspect, in the method in accordance with any one of the thirty-sixth aspect to sixty-third aspect, the low temperature storage medical container includes a syringe including the barrel defining an interior, a plunger rod configured to displace the stopper, and an outlet in fluid communication with the interior of the barrel.

In a sixty-fifth aspect, in the method in accordance with any one of the thirty-sixth aspect to the sixty-fourth aspect, the outlet is a Luer tip or a needle.

In a sixty-sixth aspect, in the method in accordance with any one of the thirty-sixth aspect to the sixty-fifth aspect, the outlet is a needle.

In a sixty-seventh aspect, in the method in accordance with any one of the thirty-sixth aspect to the sixty-sixth aspect, the low temperature storage medical container includes a therapeutic composition.

In a sixty-eighth aspect, in the method in accordance with any one of the thirty-sixth aspect to the sixty-seventh aspect, the therapeutic composition includes a vaccine composition.

In a sixty-ninth aspect, in the method in accordance with any one of the thirty-sixth aspect to the sixty-seventh aspect, the therapeutic composition includes one or more cells.

In a seventieth aspect, in the method in accordance with any one of the thirty-sixth aspect to the sixty-ninth aspect, the low temperature storage medical container is intended to be stored at temperatures of less than or equal to -60°C, such as less than or equal to -80°C, or such as less than or equal to -170°C.

### BRIEF DESCRIPTION OF THE DRAWING

Other features, embodiments and advantages of the invention will be apparent from the detailed description that follows, based on the appended drawings wherein:

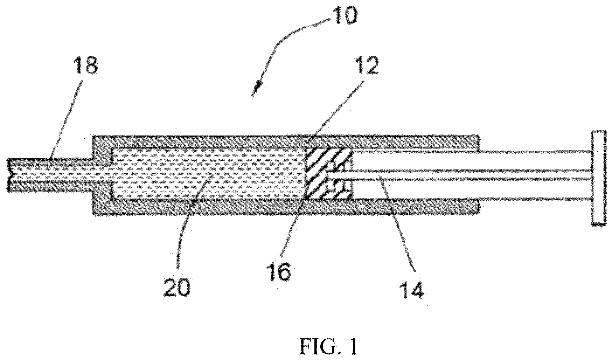

FIG. 1 shows a schematic view of a syringe.

### DESCRIPTION OF THE INVENTION

The following description is provided to enable those skilled in the art to make and use the described aspects contemplated for carrying out the disclosure. Various modifications, equivalents, variations, and alternatives, however, will remain readily apparent to those skilled in the art. Any and all such modifications, variations, equivalents, and alternatives are intended to fall within the spirit and scope of the present disclosure.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

All numbers used in the specification and claims are to be understood as being modified in all instances by the term "about". The terms "approximately", "about", and "substantially" mean a range of plus or minus ten percent of the stated value. Further, the term "substantially equal" and like terms mean that the compared values or dimensions are within a range of plus or minus ten percent of one another.

The term "at least" is synonymous with "greater than or equal to".

The term "not greater than" is synonymous with "less than or equal to".

The term "includes" is synonymous with "comprises".

The terms "first", "second", and the like are not intended to refer to any particular order or chronology, but refer to different conditions, properties, or elements.

As used herein with reference to a medical container, such as a syringe, the term "proximal" refers to an end of the apparatus farthest from the outlet, or to a direction toward the end of the apparatus farthest from the outlet. As used herein with reference to a medical container, such as a syringe, the term "distal" refers to an end of the device or apparatus closest to the outlet, or to a direction toward the end of the apparatus closest to the outlet.

As used herein, "at least one of" is synonymous with "one or more of'. For example, the phrase "at least one of A, B, and C" means any one of A, B, or C, or any combination of any two or more of A, B, or C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C.

The invention comprises, consists of, or consists essentially of the following examples of the invention, in any combination. Various examples of the invention may be discussed separately. However, it is to be understood that this is simply for ease of illustration and discussion. In the practice of the invention, one or more aspects of the invention described in one example can be combined with one or more aspects of the invention described in one or more of the other examples.

The invention is directed to a low temperature storage medical container. As used herein, a "low temperature storage medical container" is a medical container that is intended to be frozen and then stored at temperatures of less than or equal to -60°C, such as less than or equal to -80°C, or such as less than or equal to -170°C. For example, the low temperature storage medical container may be frozen and stored in liquid nitrogen having a temperature of about -196°C. The low temperature storage medical container may be thawed to room temperature, such as at least 25°C, after being frozen and stored at a temperature of less than or equal to -60°C, such as less than or equal to -80°C, or such as less than or equal to -170°C.

The low temperature storage medical container comprises a barrel comprising an inner surface. The barrel of the low temperature storage medical container may be plastic or may be glass. For example, the barrel may be plastic and may be made of polyolefins, such as high-density polyethylene, polypropylene, or cyclic polyolefins, or Crystal Clear Polymer.

The low temperature storage medical container comprises a stopper in gliding arrangement with at least a portion of the inner surface of the barrel. Stoppers, and elastomeric materials for use in the same, are known to those of skill in the art.

The stopper may be formed of a natural or synthetic rubber. For example, the rubber may be butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, and/or natural rubbers. In non-limiting aspects or embodiments, the stopper comprises butyl rubber.

The stopper may be formed of an elastomeric copolymer, including, without limitation, thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS), in which the content of styrene in the styrene block copolymer ranges from about 10% to about 70%, and preferably from about 20% to about 50%. The elastomeric composition can include, without limitation, antioxidants and/or inorganic reinforcing agents to preserve the stability of the elastomer composition, a vulcanizing agent, a vulcanizing accelerator, a vulcanizing activator, a processing aid, a filler, etc., to maintain and improve the physical properties and heat resistance of the rubber material.

The stopper may be a coated stopper or an uncoated stopper. For example, the stopper may be coated with polytetrafluoroethylene (PTFE).

The low temperature storage medical container may be a syringe. FIG. 1 shows a non-limiting embodiment or aspect of a syringe **10.** Syringe **10** includes a syringe, for example a prefilled syringe, having a barrel **12,** a plunger rod **14,** an outlet **18** in fluid communication with the interior of the barrel **20,** and a stopper **16.** Syringe barrel **12** includes a proximal end, a distal end, and a sidewall **12** therebetween defining an interior configured or adapted to contain a therapeutic composition. Plunger rod **14** is arranged at proximal end of barrel **12,** and an outlet **18** in fluid communication with the interior of the barrel **20** is arranged at distal end of barrel **12.** The outlet **18** may be a Luer tip or a stacked needle (not shown). Barrel **12** can be formed of any suitable material. In non-limiting embodiments or aspects, barrel **12** is formed of glass or a polymer, such as a plastic. Plunger rod **14** is configured to displace the stopper **16.** While not shown, syringe **10** may include safety features such as a rigid needle shield (RNS), a displaceable needle sleeve, and/or any other known features. In addition, syringe **10** may be configured for use in an auto-injector.

At least one of the inner surface of the barrel and the stopper are at least partially coated with a lubricant composition. For example, the inner surface of the barrel may be the only component of the low temperature storage medical container that is coated with the lubricant composition. Alternatively, the stopper may be the only component of the low temperature storage medical container that is coated with the lubricant composition. Alternatively, both the inner surface of the barrel and the stopper may be coated with the lubricant composition.

In non-limiting embodiments or aspects, the container may be a vial or any other type of container that may hold or may be configured to hold a therapeutic composition. In non-limiting embodiments or aspects, the container is one configured for use in a wearable injection device.

The lubricant composition of the present invention comprises at least one of the compound of Formula (I), the compound of Formula (II), or combinations thereof.

The compound of Formula (I) comprises the following structure: 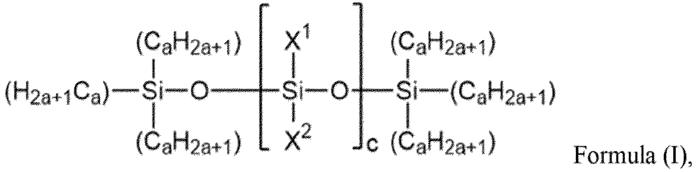 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1}, wherein b is different from a, and wherein c is an integer greater than or equal to 1.

In the compound of Formula (I), X¹ and X² may both comprise C_{b}H_{2b+1} and a and b may be greater than or equal to 2 and less than or equal to 10, such as greater than or equal to 2 and less than or equal to 5.

For example, when X¹ and X² comprise C_{b}H_{2b+1} and a and b are 2, Formula (I) comprises the following structure: 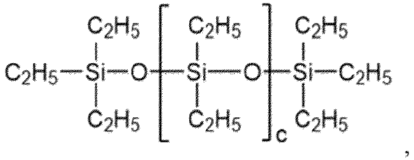 where c is greater than or equal to 1.

In the compound of Formula (I), X¹ may comprise C_{d}H_{2d}CH(C_{f}H_{2f+1})phenyl and X² may comprise CₐH₂ₐ₊₁, where a, d, and f are each independently greater than or equal to 1.

For example, when X¹ comprises C_{d}H_{2d}CH(C_{f}H_{2f+1})phenyl, where d and f are 1, and X² comprises CₐH₂ₐ₊₁, where a is 1, Formula (I) comprises the following structure: 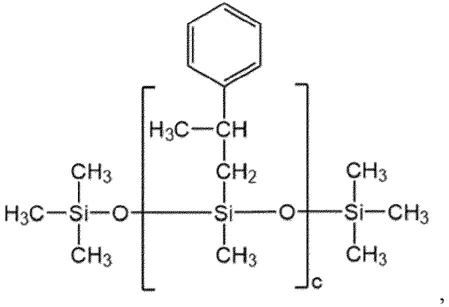 where c is greater than or equal to 1.

In the compound of Formula (I), X¹ and X² may comprise phenyl and a may be greater than or equal to 1.

For example, when X¹ and X² comprise phenyl and a is 1, Formula (I) comprises the following structure: 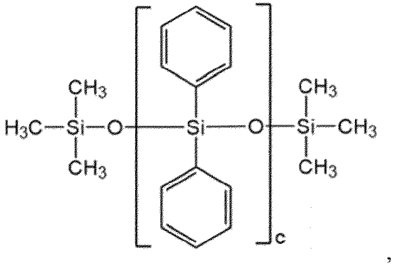 where c is greater than or equal to 1.

The compound of Formula (II) comprises the following structure: 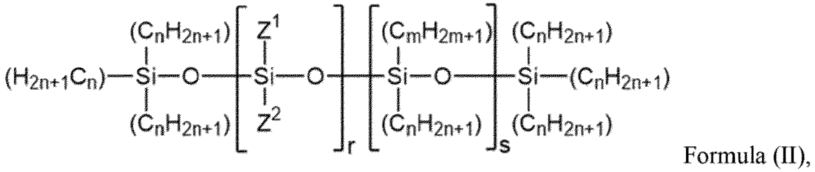 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥF_{bv+1}, wherein p and t are each independently integers greater or equal to 1, wherein v is an integer that is different from n, and wherein when Z² is CₙH₂ₙ₊₁, Z¹ is phenyl, CₚH₂ₚCH(CₜH₂ₜ₊₁)phenyl, or CᵥF₂ᵥ₊₁, wherein v is different from n, and wherein r and s are each independently an integer greater than or equal to 1.

In the compound of Formula (II), Z¹ and Z² may comprise a phenyl and m and n may each independently be greater than or equal to 1.

For example, when Z¹ and Z² comprise phenyl and m and n are 1, Formula (II) comprises the following structure: 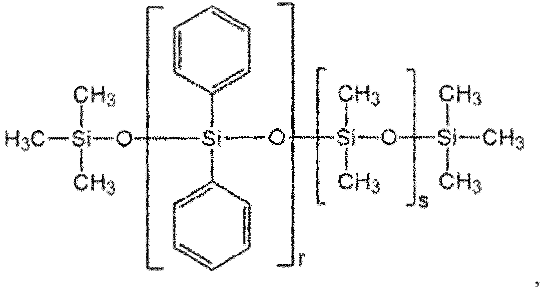 wherein r and s are each independently greater than or equal to 1.

In the compound of Formula (II), Z¹ may comprise CₚH₂ₚCH(CtH₂ₜ₊₁)phenyl and Z² may comprise CₙH₂ₙ₊₁, where p and t are each independently integers greater or equal to 1, n is 1, and m is greater than or equal to 1. For example, m may be greater than or equal to 2 and less than or equal to 12, such as greater than or equal to 2 and less than or equal to 8.

When Z¹ comprises CₚH₂ₚCH(CₜH₂ₜ₊₁)phenyl, where p and t are 1, Z² comprises CₙH₂ₙ₊₁, where n is 1, and m is 12, Formula (II) comprises the following structure: 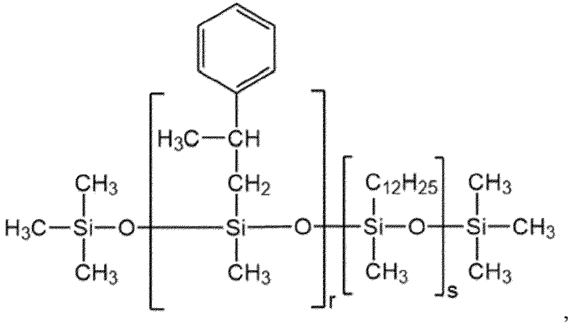 where r and s are each independently an integer greater than or equal to 1.

When Z¹ comprises CₚH₂ₚCH(CₜH₂ₜ₊₁)phenyl, where p and t are 1, Z² comprises CₙH₂ₙ₊₁, where n is 1, and m is 8, Formula (II) comprises the following structure: 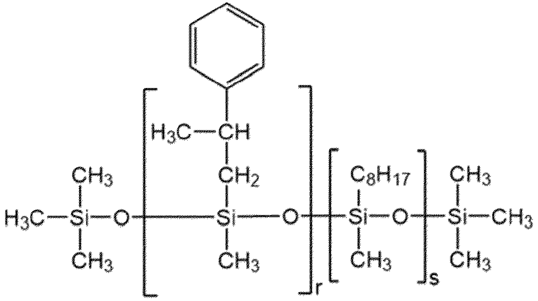 where r and s are each independently an integer greater than or equal to 1.

When Z¹ comprises CₚH₂ₚCH(CₜH₂ₜ₊₁)phenyl, where p and t are 1, Z² comprises CₙH₂ₙ₊₁, where n is 1, and m is 6, Formula (II) comprises the following structure: 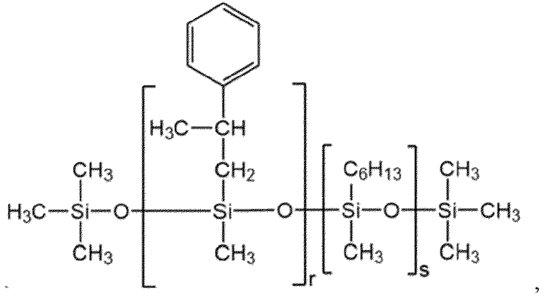 where r and s are each independently an integer greater than or equal to 1.

In the compound of Formula (II), Z¹ and Z² may comprise CᵥF₂ᵥ₊₁ where v is an integer that is different from n. For example, n may be 1 and v may be greater than or equal to 2.

In the compound of Formula (II), the compound may comprise from at least 45% to at least 85% of 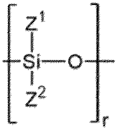 and from at least 15% to at least 55% of 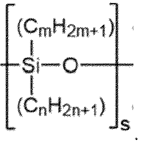 For example, the compound of Formula (II) may comprise from at least 45% to at least 55% of 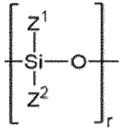 and at least 45% to at least 55% of 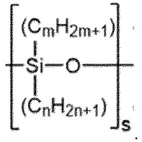 For Example, the compound of Formula (II) may comprise at least 30% to 40% of 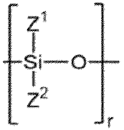 and from at least 60% to at least 70% of 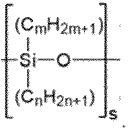 For example, the compound of Formula (II) may comprise from at least 15% to at least 25% of 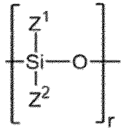 and at least 75% to at least 85% of

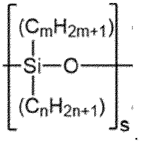

The lubricant composition may further comprise one or more small molecule surfactants, one or more an anti-nucleation agents, or combinations thereof.

For example, the lubricant composition may comprise one or small molecule surfactants, such as non-ionic small molecule surfactants. A non-limiting example of a non-ionic small molecule surfactant that may be included in the lubricant composition is polyoxyethylene sorbitan monolaurate (Tween^{®} 20).

For example, the lubricant composition may comprise one or more anti-nucleation agents, such as a surfactant (e.g., Tween^{®} 80).

The compound of Formula (I), Formula (II), or combinations thereof may be selected such that the lubricant composition comprises a viscosity of about 200 centistokes (cSt) to about 5,000 cSt, such as about 500 cSt to about 2,000 cSt.

The lubricant composition may be applied to at least one of the inner surface of the barrel and the stopper in a coating thickness of at least 1.0 nanometers (nm) or such as at least 5.0 nm.

After coating the low temperature storage medical device, the low temperature storage medical device may comprise an average lubricant composition thickness to surface roughness of at least one of the inner surface of the barrel and the stopper ratio of at least 0.001 to 3.0. The roughness of the inner surface of the barrel may be about 5 nm. The roughness of the stopper may be about 1 micron. Lower thickness lubricant layers may occur with baked lubricant of a few microns.

In reference to FIG. 1, the low temperature storage medical container may be filled with a therapeutic composition. Once the lubricant composition has been at least partially coated onto at least one of the inner surface of the barrel and the stopper, the barrel may be filled with a liquid, such as a liquid therapeutic composition, before being closed with the stopper to form the low temperature storage medical container.

The therapeutic composition may be any suitable therapeutic composition. For example, the therapeutic composition may be a vaccine composition or may comprise one or more cells.

The lubricant composition should not interact with the therapeutic composition that is intended to be filled into the low temperature storage medical container. The lubricant composition should not contain elements that could be extracted into the therapeutic composition stored in the low temperature storage medical container.

The lubricant composition, when at least partially coated onto at least one of the inner surface of the barrel and the stopper, does not crystallize during freezing, for example at temperatures of less than or equal to -60°C, such as less than or equal to -80°C, or such as less than or equal to -170°C. In addition, the lubricant composition, when at least partially coated onto at
least one of the inner surface of the barrel and the stopper, does not crystallize during thawing, where the low temperature storage medical container warms from a temperature of less than or equal to -60°C, such as less than or equal to -80°C, or such as less than or equal to -170°C to room temperature, such as 25°C.

The present invention is also directed to a method of reducing crystallization of a lubricant coating on a low temperature storage medical container during freezing and thawing. The medical container comprises a barrel comprising an inner surface; and a stopper in gliding arrangement with at least a portion of the inner surface of the barrel. The method comprises: coating a lubricant composition on at least one of the inner surface of the barrel and the stopper, the lubricant composition comprising at least one of a compound of Formula (I), a compound of Formula (II), or combinations thereof.

The compound of Formula (I) comprises the following structure: 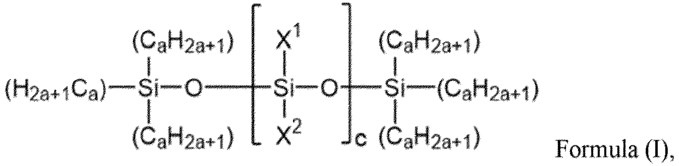 wherein a is an integer greater than or equal 1, wherein X¹ and X² are each independently phenyl, CₐH₂ₐ₊₁, C_{d}H_{2d}CH(C_{f}H_{2f+1})phenyl, or C_{b}H_{2b+1}, wherein b is an integer greater than or equal to 2, wherein d and f are each independently integers greater than or equal to 1, wherein when at least one of X¹ and X² is C_{b}H_{2b+1}, b is different from a, wherein when X² is CₐH₂ₐ₊₁, X¹ is phenyl, C_{d}H_{2d}CH(C_{f}H_{2f+1})phenyl, or C_{b}H_{2b+1}, wherein b is different from a, and wherein c is an integer greater than or equal to 1.

The compound of Formula (II) comprises: 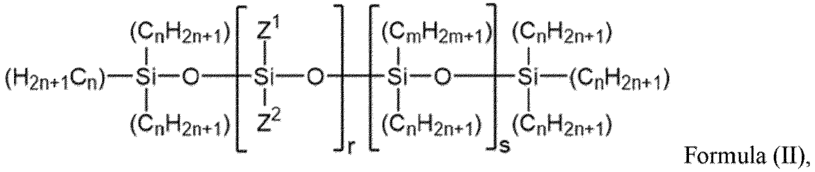 wherein n and m are each independently integers greater than or equal to 1, wherein Z¹ and Z² are each independently phenyl, CₙH₂ₙ₊₁, CₚH₂ₚCH(CₜH₂ₜ₊₁)phenyl, or CᵥF₂ᵥ₊₁, wherein p and t are each independently integers greater or equal to 1, wherein v is an integer that is different from n, and wherein when Z² is CₙH₂ₙ₊₁, Z¹ is phenyl, CₚH₂ₚCH(CₜH₂ₜ₊₁)phenyl, or CᵥH₂ᵥ₊₁, wherein v is different from n, and wherein r and s are each independently an integer greater than or equal to 1.

The low temperature storage medical containers may be any of the low temperature storage medical containers described herein. The lubricant composition and the compounds of Formula (I) and (II) may be any of the compositions and compounds described herein.

The present invention is described in the following illustrative, non-limiting examples. Numerous possible modifications and variations will be apparent to those skilled in the art.

### EXAMPLES

### Sample 1

A lubricant composition having a compound of Formula (I) was prepared. The compound had the following structure and was purchased from Gelest (APT-133; Morrisville, Pennsylvania, USA):

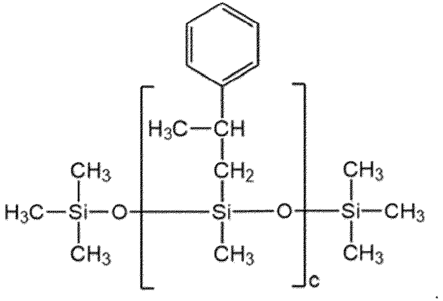

The lubricant composition of Sample 1 had a viscosity of 1,000 cSt.

### Sample 2

A lubricant composition having a compound of Formula (I) was prepared. The compound had the following structure and was purchased from Gelest (DES-T23):

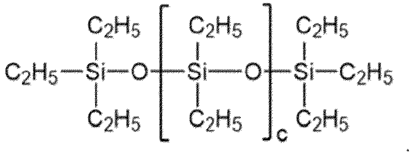

The lubricant composition of Sample 2 had a viscosity of 200 cSt to 400 cSt.

### Sample 3

A lubricant composition having a compound of Formula (II) was prepared. The compound had the following structure and was purchased from Gelest (APT-233):

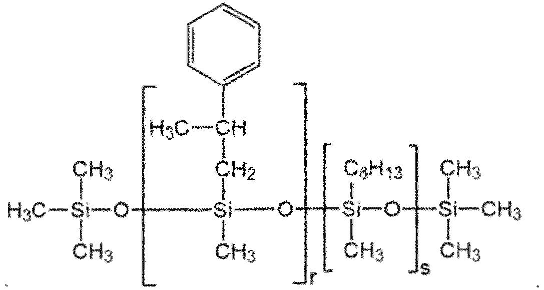

The compound had a 45% to 55% content of 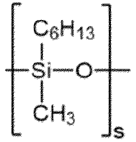 and a 45% to 55% content of 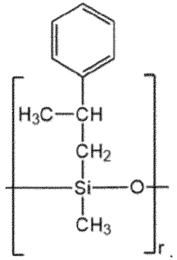 The lubricant composition of Sample 3 had a viscosity of 1,000 cSt.

### Comparative Samples 1-5

The most common silicone oil used in medical container applications, such as in syringes and stoppers, is polydimethylsiloxane, trimethylsiloxy terminated or PDMS. PDMS has the following chemical structure:

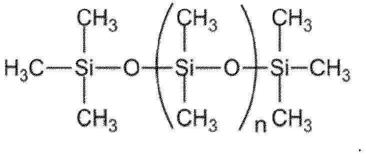

Three different viscosities (100 cSt to 12,500 cSt) and also different suppliers of PDMS oil were obtained. The samples were a PDMS oil from EWO having a viscosity of 1,000 cSt (Comparative Sample 1), a PDMS oil from Dow Corning (Midland, Michigan, USA) having a viscosity of 100 cSt (DC-360) (Comparative Sample 2), a PDMS oil from Dow Corning having a viscosity of 12,500 cSt (DC-360) (Comparative Sample 3), a PDMS oil from Nusil (Radnor, Pennsylvania USA) having a viscosity of 5,000 cSt (Med361) (Comparative Sample 4), and a PDMS oil from Gelest which had a viscosity of 1,000 cSt (DMS-T31) (Comparative Sample 5).

### Differential Scanning Calorimetry

The lubricant compositions of Samples 1-3 and Comparative Samples 1-5 were analyzed using via Differential Scanning Calorimetry (DSC; Perkin Elmer DSC 8500) in various thermal cycles down to -125°C or -130°C.

The lubricant composition of Sample 1 was held at 30°C for 1 minute, cooled from 30°C to -130°C at a rate of 3°C per minute (°C/min), held at -130°C for 2 minutes, heated from - 130°C to 0°C at a rate of 3°C/min, held at 0°C for 1 minute, cooled from 0°C to -130°C at a rate of 3°C/min, held at -130°C for 1 minute, and then heated from -130°C to 0°C at a rate of 3°C/min.

The lubricant compositions of Sample 2 was held at 30°C for 5 minutes, cooled from 30°C to -125°C at a rate of 3°C per minute (°C/min), held at -125°C for 2 minutes, heated from - 125°C to 0°C at a rate of 3°C/min, held at 0°C for 2 minutes, cooled from 0°C to -125°C at a rate of 3°C/min, held at -125°C for 2 minutes, heated from -125°C to 0°C at a rate of 3°C/min, and then held at 0°C for 1 minute.

The lubricant composition of Sample 3 was held at 30°C for 10 minutes, cooled from 30°C to -125°C at a rate of 3°C per minute (°C/min), held at -125°C for 2 minutes, heated from - 125°C to 0°C at a rate of 3°C/min, held at 0°C for 2 minutes, cooled from 0°C to -125°C at a rate of 3°C/min, held at -125°C for 2 minutes, heated from -125°C to 0°C at a rate of 3°C/min, and then held at 0°C for 1 minute.

The lubricant compositions of Comparative Samples 1-5 were held at 30°C for 1 minute, cooled from 30°C to -130°C at a rate of 3°C per minute (°C/min), held at -130°C for 2 minutes, heated from -130°C to 0°C at a rate of 3°C/min, held at 0°C for 1 minute, cooled from 0°C to -130°C at a rate of 3°C/min, held at -130°C for 1 minute, and then heated from -130°C to 0°C at a rate of 3°C/min.

Additional DSC analyses of the PDMS oils of Comparative Examples 1-5 were performed by thermal cycling up to -50°C and thermal cycling up to -85°C.

### Lubrication of Low Temperature Storage Medical Containers

### Example 1

Ten syringes were filled with Ultra Pure water and stoppered. The syringes were manually lubricated with the lubricant composition of Sample 1 and the stopper was vent-tubed in the syringe. The syringes were frozen using dry ice to a temperature of approximately -80°C and then thawed to ambient temperature.

### Example 2

Twenty-five syringes were assembled manually and filled with 1.0 mL of water purified by a Millipore Milli-Q lab water system (Milli Q water). The stoppers (butyl rubber) were manually lubricated with a PDMS oil and then placed to approximately two head space volumes: 18 syringes with a head space volume less than 0.1 mL (milliliters) and 7 syringes with head space volume between 0.2 mL and 0.25 mL. The position of each stopper's back was marked on the outside of the syringe before freezing. The syringes were cooled to liquid nitrogen temperatures in about 3 to 4 minutes.

After approximately 20 minutes in liquid nitrogen, the syringes were pulled out of the liquid nitrogen and placed in a convection oven at 30°C. The temperature of the oven overshot to 36°C causing stoppers from 2 of the large head space volume syringes to pop out.

### Differential Scanning Calorimetry Results

DSC showed, independently of the viscosity or manufacturer, that the PDMS oils of Comparative Samples 1-5 have: (1) a crystallization within the region between -50°C and -90°C; (2) the presence of a cold crystallization during thaw around -100°C; (3) a complex pattern of melting between -50°C and up to -30°C.

Thus, it was determined that the PDMS oils of Comparative Samples 1-3 show a first order thermodynamic transition that impacts the glass-stopper interfacial separation. At approximately -50°C and below the PDMS oil solidifies, having an abrupt change of specific volume (second order phase transition). At these temperatures, the rubber stopper is still with some elasticity although a high modulus. At approximately -65°C and below, the rubber stopper crosses its glass transition temperature (T_{g}) and gets rigid with a very high modulus. However, there is not significant change in the specific volume since rubber is amorphous and the transition is a first order phase transition.

The additional DSC analysis of the thermal cycle down to -50°C of the PDMS oils of Comparative Samples 1-5 showed no thermal transitions were observed during a freezing and thawing thermal cycle. The additional DSC analysis of the thermal cycle down to -85°C showed the thermal transitions, crystallization, as described above.

The cold crystallization of the PDMS oils of Comparative Samples 1-5 that was observed during thawing can occur even when the PDMS oil is cooled down to -55°C (no cooling crystallization) and the extent of the crystallization is dependent on the time that the PDMSS oil stayed at the lowest temperature as manifested by the observed melting peak.

The lubricant compositions of Samples 1-3 had significantly different thermal transitions from the PDMS oils of Comparative Samples 1-5. The lubricant compositions of Samples 1, 2, and 3 did not show a crystallization within the region between -50°C and -90°C, the presence of a cold crystallization during thaw around -100°C, or a complex pattern of melting between -50°C and up to -30°C.

The DSC results suggest that the crystallization of the lubricant composition may be prevented through the presence of very low intermolecular interactions, such as through the π to π electron cloud interactions of the phenyl side chain groups of Samples 2 and 3. In the case of Sample 1, crystallization may be prevented through stereo effects, such as through the arrangement of the ethyl groups.

### Lubrication of Low Temperature Storage Medical Containers Results

In the syringes of Example 1, which were coated with the lubricant composition of Sample 1, seven out often of the syringes did not show any carbon dioxide (CO₂) ingress.

In the syringes of Example 2, the stoppers in each syringe moved from its original position during the freezing and thawing thermal cycle. No leakage through the stopper was observed in any of the remaining five high head space volume samples. Fourteen of the low head space volume samples showed no leakage through the stopper ribs. Four of the low head space volume samples showed leakage through the stopper ribs, but it is not clear if the leakages were created by the differential warming created by holders (i.e., Styrofoam wells) and/or the temperature overshoot of the oven.

Thus, the main challenges from the freezing to -80°C and thawing cycle is not the integrity of each component of the assembled container (i.e., syringe-stopper), but rather are the dynamic changes that occur to the stopper-glass interface during the freezing and thawing thermal cycle. As the lubricant compositions of Samples 1-3 did not show either normal or cold crystallization, these lubricant compositions can be used at the glass-stopper interface and possibly up to liquid nitrogen temperature of -196°C without a risk to container enclosure integrity.

The present invention has been described with reference to specific details of particular embodiments thereof. It is not intended that such details be regarded as limitations upon the scope of the invention except insofar as and to the extent that they are included in the accompanying claims.

## Claims

1. A low temperature storage medical container comprising:
a barrel comprising an inner surface; and
a stopper in gliding arrangement with at least a portion of the inner surface of the barrel,
wherein at least one of the inner surface of the barrel and the stopper are at least partially coated with a lubricant composition, the lubricant composition comprising at least one of:
a compound of Formula (I): 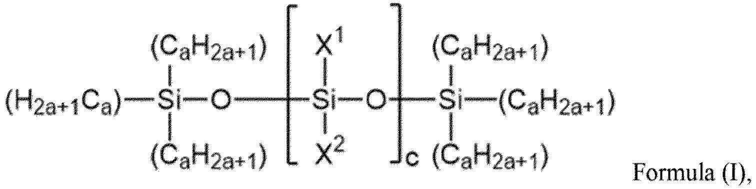
wherein a is an integer greater than or equal 1,
wherein X¹ and X² are each independently phenyl, CₐH₂ₐ₊₁, C_{d}H_{2d}CH(C_{f}H_{2f+1})phenyl, or C_{b}H_{2b+1},
wherein b is an integer greater than or equal to 2,
wherein d and f are each independently integers greater than or equal to 1,
wherein when at least one of X¹ and X² is C_{b}H_{2b+1}, b is different from a,
wherein when X² is CₐH₂ₐ₊₁, X¹ is phenyl, C_{d}H_{2d}CH(C_{f}H_{2f+1})phenyl, or C_{b}H_{2b+1}, wherein b is different from a, and
wherein c is an integer greater than or equal to 1;
a compound of Formula (II): 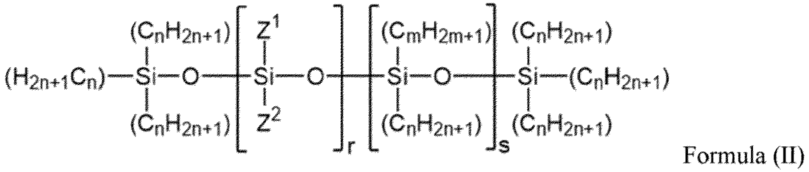
wherein n and m are each independently integers greater than or equal to 1,
wherein Z¹ and Z² are each independently phenyl, CₙH₂ₙ₊₁, CₚH₂ₚCH(CₜH₂ₜ₊₁)phenyl, or CᵥH₂ᵥ₊₁,
wherein p and t are each independently integers greater or equal to 1,
wherein v is an integer that is different from n, and
wherein when Z² is CₙH₂ₙ₊₁, Z¹ is phenyl, CₚH₂ₚCH(CₜH₂ₜ₊₁)phenyl, or CᵥH₂ᵥ₊₁, wherein v is different from n, and
wherein r and s are each independently an integer greater than or equal to 1; or
combinations thereof.

2. The low temperature storage medical container of claim 1, wherein the lubricant composition further comprises one or more small molecule surfactants, one or more an anti-nucleation agents, or combinations thereof.

3. The low temperature storage medical container of claim 1 or 2, wherein the lubricant composition comprises the compound of Formula (I) wherein a and b are greater than or equal to 2 and less than or equal to 10.

4. The low temperature storage medical container of claim 3, wherein the lubricant composition comprises: 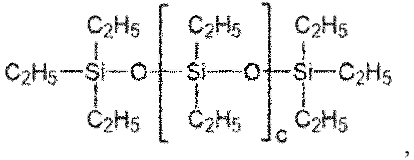 wherein c is greater than or equal to 1.

5. The low temperature storage medical container of claim 1 or 2, wherein the lubricant composition comprises: 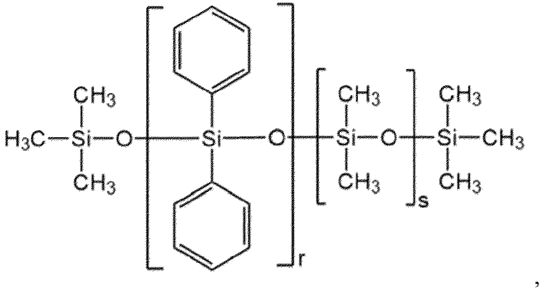 wherein r and s are each independently greater than or equal to 1.

6. The low temperature storage medical container of claim 1 or 2, wherein the lubricant composition comprises the compound of Formula (II), wherein:
Z¹ is CₚH₂ₚCH(CₜH₂ₜ₊₁)phenyl,
wherein p and t are each independently integers greater or equal to 1,
Z² is CₙH₂ₙ₊₁,
wherein n is 1,
m is greater than or equal to 1, and
r and s are each independently an integer greater than or equal to 1.

7. The low temperature storage medical container of any one of claims 1 to 6, wherein the lubricant composition comprises a viscosity of about 200 centistokes (cSt) to about 5,000 cSt.

8. The low temperature storage medical container of any one of claims 1 to 7, wherein an average lubricant composition thickness to surface roughness of at least one of the inner surface of the barrel and the stopper ratio is between 0.001 and 3.0.

9. The low temperature storage medical container of any one of claims 1 to 8, wherein the barrel is made of glass or plastic.

10. The low temperature storage medical container of any one of claims 1 to 9, wherein the stopper comprises butyl rubber.

11. The low temperature storage medical container of any one of claims 1 to 10, wherein the low temperature storage medical container comprises a syringe comprising the barrel defining an interior, a plunger rod configured to displace the stopper, and an outlet in fluid communication with the interior of the barrel.

12. The low temperature storage medical container of any one of claims 1 to 11, wherein the low temperature storage medical container comprises a therapeutic composition.

13. The low temperature storage medical container of any one of claims 1 to 12, wherein the therapeutic composition comprises a vaccine composition or one or more cells.

14. The low temperature storage medical container of any one of claims 1 to 13, wherein the low temperature storage medical container is intended to be stored at temperatures of less than or equal to -60°C.

15. A method of reducing crystallization of a lubricant coating on a low temperature storage medical container during freezing and thawing,
the medical container comprising:
a barrel comprising an inner surface; and
a stopper in gliding arrangement with at least a portion of the inner surface of the barrel,
the method comprising:
coating a lubricant composition on at least one of the inner surface of the barrel and the stopper, the lubricant composition comprising at least one of:
a compound of Formula (I): 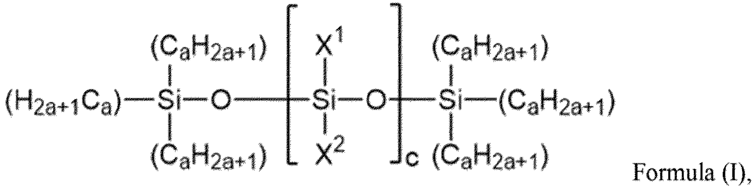
wherein a is an integer greater than or equal 1,
wherein X¹ and X² are each independently phenyl, CₐH₂ₐ₊₁, C_{d}H_{2d}CH(C_{f}H_{2f+1})phenyl, or C_{b}H_{2b+1},
wherein b is an integer greater than or equal to 2,
wherein d and f are each independently integers greater than or equal to 1,
wherein when at least one of X¹ and X² is C_{b}H_{2b+1}, b is different from a,
wherein when X² is CₐH₂ₐ₊₁, X¹ is phenyl, C_{d}H_{2d}CH(C_{f}H_{2f+1})phenyl, or C_{b}H_{2b+1}, wherein b is different from a, and
wherein c is an integer greater than or equal to 1;
a compound of Formula (II): 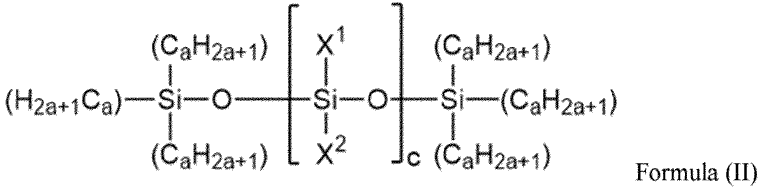
wherein n and m are each independently integers greater than or equal to 1,
wherein Z¹ and Z² are each independently phenyl, CₙH₂ₙ₊₁, CₚH₂ₚCH(CₜH₂ₜ₊₁)phenyl, or CᵥH₂ᵥ₊₁,
wherein p and t are each independently integers greater or equal to 1,
wherein v is an integer that is different from n, and
wherein when Z² is CₙH₂ₙ₊₁, Z¹ is phenyl, CₚH₂ₚCH(CₜH₂ₜ₊₁)phenyl, or CᵥF₂ᵥ₊₁, wherein v is different from n, and
wherein r and s are each independently an integer greater than or equal to 1; or
combinations thereof.
